(19) Europäisches Patentamt
European Patent Office
Office européen des brevets

(11) EP 4 549 433 A1

# (12) EUROPEAN PATENT APPLICATION

(43) Date of publication:
**07.05.2025 Bulletin 2025/19**

(21) Application number: **24207976.2**

(22) Date of filing: **22.10.2024**

(51) International Patent Classification (IPC):
***C07D 307/06*** (2006.01) ***C07D 307/08*** (2006.01)
***C07D 307/12*** (2006.01) ***C07D 307/36*** (2006.01)
***C07D 307/44*** (2006.01) ***C07C 29/132*** (2006.01)
***C07C 31/18*** (2006.01) ***C07C 31/20*** (2006.01)
***C07C 31/26*** (2006.01) ***C07H 15/04*** (2006.01)
***C01B 3/02*** (2006.01) ***C25B 1/04*** (2021.01)

(52) Cooperative Patent Classification (CPC):
(C-Sets available)
**C07D 307/44; C01B 3/02; C07C 29/132; C07D 307/06; C07D 307/08; C07D 307/12; C07D 307/36; C07H 15/04; C25B 1/04** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **31.10.2023 EP 23206867**

(71) Applicant: **BASF SE**
**67056 Ludwigshafen am Rhein (DE)**

(72) Inventors:
- **VOSSEN, Marcus**
  **67117 Limburgerhof (DE)**
- **STOCK, Christoph**
  **67056 Ludwigshafen am Rhein (DE)**
- **ERNST, Martin**
  **67056 Ludwigshafen am Rhein (DE)**
- **MOELLER, Malte**
  **67056 Ludwigshafen am Rhein (DE)**
- **PUHL, Michael**
  **67056 Ludwigshafen am Rhein (DE)**
- **KUNSMANN-KEITEL, Dagmar Pascale**
  **67056 Ludwigshafen am Rhein (DE)**
- **HARHAUSEN, Sina**
  **67056 Ludwigshafen am Rhein (DE)**
- **XIONG, Jiawen**
  **67056 Ludwigshafen am Rhein (DE)**
- **HUEFFER, Stephan**
  **67063 Ludwigshafen (DE)**
- **KRUEGER, Marco**
  **67056 Ludwigshafen am Rhein (DE)**
- **WEISS, Thomas**
  **67056 Ludwigshafen am Rhein (DE)**

(74) Representative: **BASF IP Association**
**BASF SE**
**GBI - Z078**
**67056 Ludwigshafen (DE)**

## (54) HYDROGENATION OF CARBOHYDRATES AND FURFURAL USING HYDROGEN WITH LOW DEUTERIUM CONTENT

(57) A process for the preparation of a hydrogenation product of a carbohydrate or furfural comprising the following steps:
(a) providing hydrogen with a molar share of deuterium ≤ 100 ppm, based on the total hydrogen content, by electrolysis of water using electrical power generated at least in part from non-fossil energy,
(b) at least partially hydrogenating a carbohydrate or furfural to form the corresponding hydrogenation product of the carbohydrate or furfural.

EP 4 549 433 A1

(52) Cooperative Patent Classification (CPC): (Cont.)

C-Sets
**C07C 29/132, C07C 31/18;**
**C07C 29/132, C07C 31/20;**
**C07C 29/132, C07C 31/26**

## Description

The present invention relates to the hydrogenation of carbohydrates and furfural using hydrogen. The obtained hydrogenation products (sugar alcohols and other chemicals) have a lower deuterium content, based on the total hydrogen content, compared to corresponding hydrogenation products generated by reaction with hydrogen that is produced from petrochemical processes using natural gas, condensate or petroleum oil. The invention further relates to the hydrogenation products obtained thereby as well as to their use.

In the preparation of products obtained by the hydrogenation of carbohydrates, hydrogen and in most cases a catalyst is used.

In modern industrial processes, a significant amount of the hydrogen is provided by steam reforming, thus, from natural gas. However, the petrochemical steam reforming process has its negative impacts regarding its carbon footprint including the consumption of a lot of fossil-based natural resources and energy.

It is therefore an objective of the present invention to provide environmentally friendly products obtained by the hydrogenation of carbohydrates in an environmentally friendly process for making the same, that process using as little fossil-based energy as possible.

The object is achieved by a process for the preparation of hydrogenation products of carbohydrates or furfural comprising the following steps:

(a) providing hydrogen with a molar share of deuterium ≤ 100 ppm, based on the total hydrogen content, by electrolysis of water using electrical power generated at least in part from non-fossil energy,
(b) at least partially hydrogenating a carbohydrate or furfural to form the corresponding hydrogenation product.

The object is further achieved by hydrogenation products of carbohydrates or furfural, wherein the molar share of deuterium in the hydrogen bound in the hydrogenation product as a result of step (b) of the process according to the invention is ≤ 100 ppm, preferably in the range of from 10 to 95 ppm, more preferably in the range of from 15 to 90 ppm, most preferably in the range of from 20 to 80 ppm, especially in the range of from 30 to 75 ppm, based on the total content of said hydrogen.

The molar share of deuterium in hydrogen and downstream compounds based on hydrogen is given in the present application in ppm, based on the total hydrogen content, which is the mol-ppm content of deuterium, based on the total hydrogen content (in hydrogen or in the compounds discussed, respectively).

The deuterium content of hydrogen and downstream compounds based on hydrogen is given in the present application in atom-ppm based on the total molar hydrogen content (total atoms of protium $^1H$ and deuterium $^2H$). The terms "deuterium content" and "molar share of deuterium" are used synonymously throughout the application.

In physical organic chemistry, a kinetic isotope effect is the change in the reaction rate of a chemical reaction when one of the atoms in the reactants is replaced by one of its isotopes. Formally, it is the ratio of rate constants $k_L$ / $k_H$ for the reactions involving the light ($k_L$) and the heavy ($k_H$) isotopically substituted reactants (isotopologues). This change in reaction rate is a quantum mechanical effect that primarily results from heavier isotopologues having lower vibrational frequencies compared to their lighter counterparts. In most cases, this implies a greater energetic input needed for heavier isotopologues to reach the transition state, and consequently a slower reaction rate.

Isotopic rate changes are most pronounced when the relative mass change is greatest since the effect is related to vibrational frequencies of the affected bonds. For instance, changing a hydrogen atom (H) to its isotope deuterium (D) represents a 100 % increase in mass, whereas in replacing $^{12}C$ with $^{13}C$, the mass increases by only 8 percent. The rate of a reaction involving a C-H bond is typically 6-10 times faster than the corresponding C-D bond, whereas a $^{12}C$ reaction is only 4 percent faster than the corresponding $^{13}C$ reaction.

A primary kinetic isotope effect may be found when a bond to the isotope atom is being formed or broken. A secondary kinetic isotope effect is observed when no bond to the isotope atom in the reactant is broken or formed. Secondary kinetic isotope effects tend to be much smaller than primary kinetic isotope effects; however, secondary deuterium isotope effects can be as large as 1.4 per deuterium atom.

Step (a)

Step (a) concerns the electrolysis of water using electrical power generated at least in part from non-fossil energy.

Electrolysis of water is an environmentally friendly method for production of hydrogen because it uses renewable $H_2O$ and produces only pure oxygen as by-product. Additionally, water electrolysis utilizes direct current (DC) from sustainable energy resources, for example solar, wind, hydropower and biomass.

It is observed that by electrolysis of water, the deuterium atom content of the hydrogen is lower than in the hydrogen generated petrochemically, for example as contained in synthesis gas, in general ≤ 100 ppm, preferably in general ≤ 90 ppm, for example from 30 to 75 ppm. The deuterium atom content in electrolytically produced hydrogen may

be as low as 10 ppm. The deuterium is mainly present in the form of D-H rather than $D_2$.

One suitable water electrolysis process is alkaline water electrolysis. Hydrogen production by alkaline water electrolysis is a well-established technology up to the megawatt range for a commercial level. In alkaline water electrolysis initially at the cathode side two water molecules of alkaline solution (KOH/NaOH) are reduced to one molecule of hydrogen ($H_2$) and two hydroxyl ions (OH-). The produced $H_2$ emanates from the cathode surface in gaseous form and the hydroxyl ions (OH-) migrate under the influence of the electrical field between anode and cathode through the porous diaphragm to the anode, where they are discharged to half a molecule of oxygen ($O_2$) and one molecule of water ($H_2O$). Alkaline electrolysis operates at lower temperatures such as 30-80°C with alkaline aqueous solution (KOH/NaOH) as the electrolyte, the concentration of the electrolyte being about 20% to 30 %. The diaphragm in the middle of the electrolysis cell separates the cathode and anode and also separates the produced gases from their respective electrodes, avoiding the mixing of the produced gases. However, alkaline electrolysis has negative aspects such as limited current densities (below 400 mA/$cm^2$), low operating pressure and low energy efficiency.

In one preferred embodiment of the inventive process, hydrogen is provided by polymer electrolyte membrane water electrolysis. Variants of polymer electrolyte membrane water electrolysis are proton exchange membrane water electrolysis (PEMWE) and anion exchange membrane water electrolysis (AEMWE).

PEM water electrolysis was developed to overcome the drawbacks of alkaline water electrolysis. PEM water electrolysis technology is similar to the PEM fuel cell technology, where solid polysulfonated membranes (Nation®, fumapem®) are used as an electrolyte (proton conductor). These proton exchange membranes have many advantages such as low gas permeability, high proton conductivity ($0.1 \pm 0.02$ S $cm^{-1}$), low thickness (20-300 μm), and allow high-pressure operation. In terms of sustainability and environmental impact, PEM water electrolysis is one of the most favorable methods for conversion of renewable energy to highly pure hydrogen. PEM water electrolysis has great advantages such as compact design, high current density (above 2 A $cm^{-2}$), high efficiency, fast response, operation at low temperatures (20-80°C) and production of ultrapure hydrogen. The state-of-the-art electrocatalysts for PEM water electrolysis are highly active noble metals such as Pt/Pd for the hydrogen evolution reaction (HER) at the cathode and $IrO_2/RuO_2$ for the oxygen evolution reaction (OER) at the anode.

One of the largest advantages of PEM water electrolysis is its ability to operate at high current densities. This can result in reduced operational costs, especially for systems coupled with very dynamic energy sources such as wind and solar power, where sudden spikes in energy output would otherwise result in uncaptured energy. The polymer electrolyte allows the PEM water electrolyzer to operate with a very thin membrane (ca. 100-200 μm) while still allowing high operation pressure, resulting in low ohmic losses, primarily caused by the conduction of protons across the membrane (0.1 S/cm), and a compressed hydrogen output.

The PEM water electrolyzer utilizes a solid polymer electrolyte (SPE) to conduct protons from the anode to the cathode while insulating the electrodes electrically. Under standard conditions the enthalpy required for the formation of water is 285.9 kJ/mol. One portion of the required energy for a sustained electrolysis reaction is supplied by thermal energy and the remainder is supplied through electrical energy.

The half reaction taking place on the anode side of a PEM water electrolyzer is commonly referred to as the Oxygen Evolution Reaction (OER). Here the liquid water reactant is supplied to a catalyst where it is oxidized to oxygen, protons and electrons.

The half reaction taking place on the cathode side of a PEM water electrolyzer is commonly referred to as the Hydrogen Evolution Reaction (HER). Here the protons that have moved through the membrane are reduced to gaseous hydrogen.

PEMs can be made from either pure polymer membranes or from composite membranes, where other materials are embedded in a polymer matrix. One of the most common and commercially available PEM materials is the fluoropolymer PFSA, or Nation®, a DuPont product. While Nation® is an ionomer with a perfluorinated backbone like Teflon, there are many other structural motifs used to make ionomers for proton-exchange membranes. Many use polyaromatic polymers, while others use partially fluorinated polymers.

An overview over hydrogen production by PEM water electrolysis is given in S. Kumar and V. Himabindu, Material Science for Energy Technologies 2 (2019), pp. 4442 - 4454.

An overview over hydrogen production by anion exchange membrane water electrolysis is given in H. A. Miller et al., Sustainable Energy Fuels, 2020, 4, pp. 2114 - 2133.

K. Harada et al., International Journal of Hydrogen Energy 45 (2020), pp. 31389 - 31395 report a deuterium depletion by a factor from 2 to 3 in polymer electrolyte membrane water electrolysis. The separation factor β

$$\beta = ([H]/[D])_{gas} / ([H]/[D])_{liquid}$$

where "gas" is the evolved gas and "liquid" is water before the electrolysis was found to be between 2 and 3 at current densities of from 1.0 to 2.0 A $cm^{-2}$, corresponding to a stoichiometric number A of between 4 and 9 at the given water mass flow in the anode. The stoichiometric number A is defined as follows:

$$\lambda = V \times \rho / (J/2F \times 60 \times M_{H2O})$$

where V (mL $min^{-1}$) is the water mass flow in the anode, F is the Faraday constant, J is electrolysis current (A), ρ is the density of water (g $mL^{-1}$) and $M_{H2O}$ (g $mol^{-1}$) is the molar weight of water. A stoichiometric number A of 10 means that 10 times the amount of fresh water than can be theoretically consumed by electrolysis at the given electrolysis current is supplied to the anode.

H. Sato et al., International Journal of Hydrogen Energy 46 (2021), pp. 33 689 - 33 695, report for anion exchange membrane water electrolysis that deuterium concentration in the evolving hydrogen gas is diluted by approximately 1/5 against the feed water, at A = 4.

Hence, deuterium in the evolving hydrogen gas can easily be depleted by a factor of from 2 to 5 with regard to feed water in polymer electrolyte membrane water electrolysis. Depending on the electrolysis conditions (water flow, current density), even higher depletion factors are possible. Since the average deuterium content of water is about 150 ppm, based on the total hydrogen content, hydrogen provided in step (a) of the inventive process may have a deuterium content of from 30 to 75 ppm, based on the total hydrogen content, or even lower.

The electrical power is generated at least in part from non-fossil, renewable resources. In other words, part of the electrical power can still be produced from fossil fuels, preferably from natural gas, since combustion of natural gas causes much lower carbon dioxide emission per Megajoule of electrical energy produced than combustion of coal. However, the portion of electrical energy produced from fossil fuels should be as low as possible, preferably ≤ 50%, preferably ≤ 30%, most preferably ≤ 20%.

The electrical power from non-fossil resources used in water electrolysis according to the invention can be generated by nuclear energy. Nuclear energy is considered renewable by the European Commission, as long as certain preconditions (inter alia safe long-term storage of nuclear waste) are fulfilled.

The electrical power from non-fossil resources used in water electrolysis according to the invention is preferably generated from wind power, solar energy, biomass, hydropower and geothermal energy.

In one preferred embodiment of the inventive process, the electrical power used in water electrolysis is generated from hydropower. There are many forms of hydropower. Traditionally, hydroelectric power comes from constructing large hydroelectric dams and reservoirs. Small hydro systems are hydroelectric power installations that typically produce up to 50 MW of power. They are often used on small rivers or as a low-impact development on larger rivers. Run-of-the-river hydroelectricity plants derive energy from rivers without the creation of a large reservoir. The water is typically conveyed along the side of the river valley (using channels, pipes and/or tunnels) until it is high above the valley floor, whereupon it can be allowed to fall through a penstock to drive a turbine.

Wave power, which captures the energy of ocean surface waves, and tidal power, converting the energy of tides, are two forms of hydropower with future potential.

In one further preferred embodiment of the inventive process, the electrical power used in electrolysis is generated at least in part from geothermal energy. Geothermal energy is the heat that comes from the sub-surface of the earth. It is contained in the rocks and fluids beneath the earth's crust and can be found as far down to the earth's hot molten rock, magma.

To produce power from geothermal energy, wells are dug a mile deep into underground reservoirs to access the steam and hot water there, which can then be used to drive turbines connected to electricity generators. There are three types of geothermal power plants; dry steam, flash and binary. Dry steam is the oldest form of geothermal technology and takes steam out of the ground and uses it to directly drive a turbine. Flash plants use high-pressure hot water into cool, low-pressure water whilst binary plants pass hot water through a secondary liquid with a lower boiling point, which turns to vapor to drive the turbine.

In one further preferred embodiment of the inventive process, the electrical power used in water electrolysis is generated from wind power. Wind power can be used to run wind turbines. Modern utility-scale wind turbines range from around 600 kW to 9 MW of rated power. The power available from the wind is a function of the cube of the wind speed, so as wind speed increases, power output increases up to the maximum output for the particular turbine. Areas where winds are stronger and more constant, such as offshore and high-altitude sites, are preferred locations for wind farms.

In one further preferred embodiment of the inventive process, the electrical power used in water electrolysis is generated from solar power, particularly preferred from photovoltaic systems. A photovoltaic system converts light into electrical direct current (DC) by taking advantage of the photoelectric effect. Concentrated solar power (CSP) systems use lenses or mirrors and tracking systems to focus a large area of sunlight into a small beam. CSP-Stirling has by far the highest efficiency among all solar energy technologies.

In one further preferred embodiment of the inventive process, the electrical power used in water electrolysis is generated from biomass. Biomass is biological material derived from living, or recently living organisms. It most often refers to plants or plant-derived materials which are specifically called lignocellulosic biomass. As an energy source, biomass can either be used directly via combustion to produce heat or electricity, or indirectly after converting it to various forms of biofuel. Conversion of biomass to biofuel can be achieved by different methods which are broadly classified into:

thermal, chemical, and biochemical methods. Wood was the largest biomass energy source as of 2012; examples include forest residues - such as dead trees, branches and tree stumps -, yard clippings, wood chips and even municipal solid waste. Industrial biomass can be grown from numerous types of plants, including miscanthus, switchgrass, hemp, corn, poplar, willow, sorghum, sugarcane, bamboo, and a variety of tree species, ranging from eucalyptus to oil palm (palm oil).

Plant energy is produced by crops specifically grown for use as fuel that offer high biomass output per hectare with low input energy. The grain can be used for liquid transportation fuels while the straw can be burned to produce heat or electricity. Biomass can be converted to other usable forms of energy such as methane gas or transportation fuels such as ethanol and biodiesel. Rotting garbage, and agricultural and human waste, all release methane gas - also called landfill gas or biogas. Crops, such as corn and sugarcane, can be fermented to produce the transportation fuel, ethanol. Biodiesel, another transportation fuel, can be produced from left-over food products such as vegetable oils and animal fats.

Step (b)

In step (b), a carbohydrate or furfural is hydrogenated using the hydrogen provided in step (a) to form the corresponding product obtained by the hydrogenation of the carbohydrate or of furfural.

Overview of carbohydrates

A carbohydrate is a biomolecule consisting of carbon (C), hydrogen (H) and oxygen (O) atoms, usually with a hydrogen-oxygen atom ratio of 2:1 (as in water) and thus with the empirical formula $C_m(H_2O)_n$ (where m may or may not be different from n), which does not mean the H has covalent bonds with O (for example with $CH_2O$, H has a covalent bond with C but not with O). However, not all carbohydrates conform to this precise stoichiometric definition (e.g., uronic acids, deoxy sugars such as fucose), nor are all chemicals that do conform to this definition automatically classified as carbohydrates (e.g. formaldehyde and acetic acid).

The term is most common in biochemistry, where it is a synonym of saccharide, a group that includes sugars, starch, and cellulose. The saccharides are divided into four chemical groups: monosaccharides, disaccharides, oligosaccharides, and polysaccharides. Monosaccharides and disaccharides, the smallest (lower molecular weight) carbohydrates, are commonly referred to as sugars.

Starch is a polysaccharide and is abundant in cereals (wheat, maize, rice), potatoes, and processed food based on cereal flour, such as bread, pizza or pasta. Sugars appear in human diet mainly as table sugar (sucrose, extracted from sugarcane or sugar beets), lactose (abundant in milk), glucose and fructose, both of which occur naturally in honey, many fruits, and some vegetables. Table sugar, milk, or honey are often added to drinks and many prepared foods such as jam, biscuits and cakes.

Cellulose, a polysaccharide found in the cell walls of all plants, is one of the main components of insoluble dietary fiber. Although it is not digestible by humans, cellulose and insoluble dietary fiber generally help maintain a healthy digestive system by facilitating bowel movements. Other polysaccharides contained in dietary fiber include resistant starch and inulin, which feed some bacteria in the microbiota of the large intestine and are metabolized by these bacteria to yield short-chain fatty acids.

Lignocellulosic biomass mainly contains cellulose, hemicellulose, and lignin, which constitute 40-50%, 25-35%, and 15-20% of lignocellulose, respectively.

In scientific literature, the term "carbohydrate" has many synonyms, like "sugar" (in the broad sense), "saccharide", "ose", "glucide", "hydrate of carbon" or "polyhydroxy compounds with aldehyde or ketone".

Natural saccharides are generally built of simple carbohydrates called monosaccharides with general formula $(CH_2O)_n$ where n is three or more. A typical monosaccharide has the structure $H\text{-}(CHOH)_x(C{=}O)\text{-}(CHOH)_y\text{-}H$, that is, an aldehyde or ketone with many hydroxyl groups added, usually one on each carbon atom that is not part of the aldehyde or ketone functional group. Examples of monosaccharides are glucose, fructose, and glyceraldehydes.

The open-chain form of a monosaccharide often coexists with a closed ring form where the aldehyde/ketone carbonyl group carbon (C=O) and hydroxyl group (-OH) react forming a hemiacetal with a new C-O-C bridge. Monosaccharides can be linked together into what are called polysaccharides (or oligosaccharides) in a large variety of ways. Many carbohydrates contain one or more modified monosaccharide units that have had one or more groups replaced or removed. For example, deoxyribose, a component of DNA, is a modified version of ribose; chitin is composed of repeating units of N-acetyl glucosamine, a nitrogen-containing form of glucose.

Carbohydrates may be classified according to their degree of polymerization, and may be divided initially into three principal groups, namely sugars, oligosaccharides and polysaccharides, as exemplified in Table 1.

Table 1: Typical carbohydrates

| Class (degree of polymerization) | Subgroup | Components |
|---|---|---|
| Sugars (1-2) | Monosaccharides | Glucose, galactose, fructose, xylose, mannose, sorbose, rhamnose, fucose |
| | Disaccharides | Sucrose, lactose, maltose, isomaltulose, trehalose, cellobiose |
| | | |
| Oligo-saccharides (3-9) | Oligo-saccharides | Maltodextrins, raffinose, stachyose, fructo-oligosaccharides, starch oligomers, xyloglucan oligomers, alginate oligomers |
| Poly-saccharides (>9) | Starch | Amylose, amylopectin, modified starches |
| | Non-starch polysaccharides | Glycogen, Cellulose, Hemicellulose, Pectins, Hydro-colloids, xyloglucan, alginat |

Types of Monosaccharides: Monosaccharides are classified according to three different characteristics: the placement of its carbonyl group, the number of carbon atoms it contains, and its chiral handedness. If the carbonyl group is an aldehyde, the monosaccharide is an aldose; if the carbonyl group is a ketone, the monosaccharide is a ketose. Monosaccharides with three carbon atoms are called trioses, those with four are called tetroses, five are called pentoses, six are hexoses, and so on. These two systems of classification are often combined. For example, glucose is an aldohexose (a six-carbon aldehyde), ribose is an aldopentose (a five-carbon aldehyde), and fructose is a ketohexose (a six-carbon ketone).

The aldehyde or ketone group of a straight-chain monosaccharide will react reversibly with a hydroxyl group on a different carbon atom to form a hemiacetal or hemiketal, forming a heterocyclic ring with an oxygen bridge between two carbon atoms. Rings with five and six atoms are called furanose and pyranose forms, respectively, and exist in equilibrium with the straight-chain form.

Deoxy sugars are sugars that have had a hydroxyl group replaced with a hydrogen atom.

Examples include:

- Deoxyribose, or 2-deoxy-D-ribose, a constituent of DNA
- Fucose, or 6-deoxy-L-galactose, main component of fucoidan of brown algae, and present in N-linked glycans
- Fuculose, or 6-deoxy-L-tagatose, one of the important components of avian influenza virus particles
- Rhamnose, or 6-deoxy-L-mannose, present in plant glycosides

Dideoxy sugars: some biologically important dideoxy sugars, sugars that have had two hydroxyl groups replaced with hydrogen atoms, include colitose and abequose.

Hexoses: in chemistry, a hexose is a monosaccharide (simple sugar) with six carbon atoms. The chemical formula for all hexoses is $C_6H_{12}O_6$, and their molecular weight is 180.156 g/mol.

Hexoses exist in two forms, open-chain or cyclic, that easily convert into each other in aqueous solutions. The open-chain form of a hexose, which usually is favored in solutions, has the general structure $H-(CHOH)_n-1-C(=O)-(CHOH)_6-n-H$, where n is 1, 2, 3, 4, 5. Namely, five of the carbons have one hydroxyl functional group (-OH) each, connected by a single bond, and one has an oxo group (=O), forming a carbonyl group (C=O). The remaining bonds of the carbon atoms are satisfied by seven hydrogen atoms. The carbons are commonly numbered 1 to 6 starting at the end closest to the carbonyl.

Aldohexoses and Ketohexoses: when the carbonyl is in position 1, forming a formyl group (-CH=O), the sugar is called an aldohexose, a special case of aldose. Aldohexoses are named Allose, Altrose, Glucose, Mannose, Gulose, Idose, Galactose and Talose. If the carbonyl position is 2 or 3, the sugar is a derivative of a ketone, and is called a ketohexose, a special case of ketose; specifically, an n-ketohexose. The important ketohexoses are the 2-ketohexoses, and the most important 2-ketose is fructose. Besides the 2-ketoses, there are only the 3-ketoses, and they do not exist in nature, although at least one 3-ketohexose has been synthesized, with great difficulty. The 2-ketohexoses isomers are: Psicose, Fructose, Sorbose, and Tagatose. The term "hexose" is assumed to include deoxyhexoses, such as fucose and rhamnose: compounds with general formula $C_6H_{12}O_6$-y that can be described as derived from hexoses by replacement of one or more hydroxyl groups with hydrogen atoms.

Pentoses: a pentose is a monosaccharide (simple sugar) with five carbon atoms. The chemical formula of many

pentoses is $C_5H_{10}O_5$, and their molecular weight is 150.13 g/mol.

Ribose is a constituent of RNA, and the related molecule, deoxyribose, is a constituent of DNA.

Like some other monosaccharides, pentoses exist in two forms, open-chain (linear) or closed-chain (cyclic), that easily convert into each other in water solutions. The linear form of a pentose, which usually exists only in solutions, has an open-chain backbone of five carbons. Four of these carbons have one hydroxyl functional group (-OH) each, connected by a single bond, and one has an oxygen atom connected by a double bond (=O), forming a carbonyl group (C=O). The remaining bonds of the carbon atoms are satisfied by six hydrogen atoms. Thus the structure of the linear form is $H-(CHOH)_x-C(=O)-(CHOH)_4-x-H$, where x is 0, 1, or 2.

Aldopentoses and Ketopentoses: the aldopentoses are a subclass of the pentoses which, in the linear form, have the carbonyl at carbon 1, forming an aldehyde derivative with structure $H-C(=O)-(CHOH)_4-H$. The most important example is ribose. The ketopentoses instead have the carbonyl at positions 2 or 3, forming a ketone derivative with structure $H-CHOH-C(=O)-(CHOH)_3-H$ (2-ketopentose) or $H-(CHOH)_2-C(=O)-(CHOH)_2-H$ (3-ketopentose). The latter is not known to occur in nature and are difficult to synthesize. The term "pentose" is assumed to include deoxypentoses, such as deoxyribose: compounds with general formula $C_5H_{10}O_5$-y that can be described as derived from pentoses by replacement of one or more hydroxyl groups with hydrogen atoms.

Types of Disaccharides: two joined monosaccharides are called a disaccharide, the simplest kind of polysaccharide. Examples include sucrose and lactose. They are composed of two monosaccharide units bound together by a covalent bond known as a glycosidic linkage formed via a dehydration reaction, resulting in the loss of a hydrogen atom from one monosaccharide and a hydroxyl group from the other. The formula of unmodified disaccharides is $C_{12}H_{22}O_{11}$.

Sucrose is the most abundant disaccharide, and the main form in which carbohydrates are transported in plants. It is composed of one D-glucose molecule and one D-fructose molecule.

Lactose, a disaccharide composed of one D-galactose molecule and one D-glucose molecule, occurs naturally in mammalian milk. Other notable disaccharides include maltose (two D-glucoses linked $\alpha$-1,4) and cellobiose (two D-glucoses linked $\beta$-1,4). Disaccharides can be classified into two types: reducing and non-reducing disaccharides. If the functional group is present in bonding with another sugar unit, it is called a reducing disaccharide or biose.

The term "sugar" or "sugars" is frequently used for specific carbohydrates (monosaccharides, disaccharides and their mixtures) and depending on its origin, quality and composition has many synonyms for example: Agave syrup, Arabinose, Barbados sugar, Barley malt syrup, barley malt, Barley sugar, Beet sugar, Birch syrup, Brown sugar, Buttered syrup, Cane sugar (cane juice, cane juice crystals), Caramel, Carob syrup, Caster sugar, Coconut sugar, Confectioner's sugar, Corn sugar, Corn syrup, Date sugar, Dehydrated cane juice, Demerara sugar, Dextrin, Dextrose, Disaccharide, Evaporated cane juice, Free sugar, Fructose, Fruit juice, Fruit juice concentrate, Fucose, Galactose, Glucose, glucose solids, Golden syrup, golden sugar, Grape sugar, grape juice, High fructose corn syrup (HFCS), High maltose corn syrup, Honey, Inverted sugar syrup, Jaggery, Lactose, Malt extract, Maltose, Maltodextrin, Maltol, Mannose, Maple sugar, Maple syrup, Molasses (from sugar beets), Molasses (from sugar cane), Monosaccharide, Muscovado, Non-centrifugal cane sugar, Palm sugar, Panela, Penuche, Powdered sugar, Raw sugar, Refiner's sugar, Ribose, Rice syrup, Rhamnose, Saccharose, Sorghum syrup, Sucrose, Sugarcane, Sweet sorghum, Syrup, Table syrup, Treacle, Trehalose, Yellow sugar, Xylose

## Hydrogenation of carbohydrates

Products generated by the hydrogenation of carbohydrates are sugar alcohols and other downstream products. Sugar alcohols (also called polyhydric alcohols, polyalcohols, alditols or glycitols) are organic compounds, typically derived from sugars, containing one hydroxyl group (-OH) attached to each carbon atom. Since they contain multiple -OH groups, they are classified as polyols.

Sugar alcohols can be and often are produced from renewables. Particular feedstocks are mono- and disaccharides, starch, cellulose and hemicellulose and the main conversion technologies are hydrogenolysis, i.e. the cleavage of CO bonds, and hydrogenation of C=O double bonds, both using $H_2$ as the reagent. Hydrogenolysis converts polymers to smaller molecules. Hydrogenation converts sugars to sugar alcohols.

Both disaccharides and monosaccharides can form sugar alcohols; however, sugar alcohols derived from disaccharides (e.g. maltitol and lactitol) are not entirely hydrogenated because only one aldehyde group is available for reduction.

Sugar alcohols are used widely in the food industry as thickeners and sweeteners and in other industries for different uses. In commercial foodstuffs, sugar alcohols are commonly used in place of table sugar (sucrose), often in combination with high-intensity artificial sweeteners, in order to offset their low sweetness. Xylitol and sorbitol are popular sugar alcohols in commercial foods. For a review on sugar alcohols see H. Schiweck et al. in "Sugar Alcohols". Ullmann's Encyclopedia of Industrial Chemistry (2012) Weinheim: Wiley-VCH, doi:10.1002/14356007.a25_413.pub3. ISBN 978-3-527-30673-2; or Ruppert, A. et al., in "Hydrogenolysis Goes Bio: From Carbohydrates and Sugar Alcohols to Platform Chemicals"; Angewandte Chemie International Edition. (2012) 51 (11): 2564-2601, doi: 1 0.1 002/anie.2011

05125. PMID 22374680.

Many hydrogenation reactions of carbohydrates and other downstream products are using catalysts. A review of catalysts used for hydrogenation is given in C.Stock (2023) Hydrogenation and Dehydrogenation; Chapter 1.3: "Catalysts" in Ullmann's Encyclopedia of Industrial Chemistry (https://doi.org/10.1002/14356007.a13_487.pub3). Preferred carbohydrates to be hydrogenated in step (b) contain a total of from 1 to 20 monosaccharide units or a total of from 3 to 120 carbon atoms.

More preferred carbohydrates to be hydrogenated in step (b) contain a total of from 1 to 10 monosaccharide units or a total of from 3 to 60 carbon atoms.

Most preferred carbohydrates to be hydrogenated in step (b) contain a total of from 1 to 5 monosaccharide units or a total of from 3 to 30 carbon atoms.

Particularly preferred carbohydrates to be hydrogenated in step (b) are monosaccharides and disaccharides to give sugar alcohols.

Particularly preferred hydrogenations are hydrogenation reactions (1) to (15):

(1) Hydrogenation of glucose to sorbitol

Sorbitol also may be synthesized through a catalytic hydrogenation of d-glucose to form d-sorbitol. This reaction has a 100% yield of d-sorbitol when d-glucose is reacted with hydrogen in water at 120 degrees Celsius for 1 hour (India Patent WO2017/60922. April 13, 2017).

(2) Hydrogenation of mannose to mannitol

Mannitol is obtained by hydrogenation of sugars, using Raney nickel catalysts. [see H. Schiweck et al. in "Sugar Alcohols". Ullmann's Encyclopedia of Industrial Chemistry (2012) Weinheim: Wiley-VCH.

doi:10.1002/14356007.a25_413.pub3.]

(3) Hydrogenation of fructose to mannitol

Mannitol is commonly produced via the hydrogenation of fructose, which is formed from either starch or sucrose according to Kearsley MW, Deis RC (2006). "Sorbitol and Mannitol", Sweeteners and Sugar Alternatives in Food Technology, Wiley-Blackwell. pp. 249-261.

(4) Hydrogenation of xylose to xylitol

Purified xylose is catalytically hydrogenated into xylitol using a Raney nickel catalyst.

See H. Schiweck et al. in "Sugar Alcohols". Ullmann's Encyclopedia of Industrial Chemistry (2012) Weinheim: Wiley-VCH. doi:10.1002/14356007.a25_413.pub3.

(5) Hydrogenation of isomaltulose to isomalt

Isomalt is manufactured in a two-stage process in which sucrose (typically derived from beet sugar) is first transformed into isomaltulose using the bacterial enzyme isomaltulose synthase. The isomaltulose is then hydrogenated using a Raney nickel catalyst, producing 1-O-α-D-glucopyranosido-D-mannitol (1,1-GPM) and 6-O-α-D-glucopyranosido-D-sorbitol (1,6-GPS). The product consists of a mixture of anhydrous and dihydrated 1,1-GPM and anhydrous 1,6-GPS. (see: 604. Isomalt (WHO Food Additives Series 20)". INCHEM. Retrieved 2017-09-28 and H. Schiweck et al. in "Sugar Alcohols". Ullmann's Encyclopedia of Industrial Chemistry (2012) Weinheim: Wiley-VCH. doi:10.1002/14356007.a25_413.pub3.).

(6) Hydrogenation of maltose to maltitol

Maltitol is a disaccharide produced by hydrogenation of maltose obtained from starch.

(see: Application A537 - Reduction in the energy factor assigned to Maltitol: Final Assessment Report (PDF), Food Standards Australia New Zealand, 5 October 2005, retrieved 27 January 2014)

(7) Hydrogenation of lactose to lactitol

Lactitol is produced by hydrogenation of lactose using Raney nickel catalyst. The product can be obtained as an anhydrous, monohydrate, or dihydrate. Two manufacturers, Danisco and Purac Biochem, produce about 10,000 tons/y

(see: H. Schiweck et al. in "Sugar Alcohols". Ullmann's Encyclopedia of Industrial Chemistry (2012) Weinheim: Wiley-VCH. doi:10.1002/14356007.a25_413.pub3.)

(8) Hydrogenation of starch hydrolysates

Hydrogenated starch hydrolysates (HSHs), also known as polyglycitol syrup (INS 964), are mixtures of several sugar alcohols (a type of sugar substitute). Hydrogenated starch hydrolysates were developed by the Swedish company Lyckeby Starch in the 1960s (Altschul, Aaron M (1993-03-12). Low-Calorie Foods Handbook. CRC Press. ISBN 9780824788124).

Hydrogenated starch hydrolysates are produced by the partial hydrolysis of starch - most often corn starch, but also potato starch or wheat starch. This creates dextrins (glucose and short glucose chains). The hydrolyzed starch (dextrin) then undergoes hydrogenation to convert the dextrins to sugar alcohols.

Because in HSHs the starch is not completely hydrolyzed, a mixture of sorbitol, maltitol, and longer chain hydrogenated saccharides (such as maltotriitol) is produced. When no single polyol is dominant in the mix, the generic name hydrogenated starch hydrolysates is used. However, if 50% or more of the polyols in the mixture are of one type, it can be labeled as "sorbitol syrup", or "maltitol syrup", etc.

(9) - (15) Hydrogenation of furfural

Furfural is a promising renewable platform compound derived from carbohydrates such as lignocellulosic biomass that can be further converted to biofuels and biochemicals. Furfural may be obtained by the acid catalyzed dehydration of 5-carbon sugars (pentoses), particularly xylose as described in Cai, Charles M et al. in "Integrated furfural production as a renewable fuel and chemical platform from lignocellulosic biomass". Journal of Chemical Technology & Biotechnology (2014) 89: 2-10. doi:10.1002/jctb.4168. These sugars may be obtained from pentosans obtained from hemicellulose present in lignocellulosic biomass.

Furfural can be hydrogenated to (9) furfurylalcohol; (10) 2-methylfuran; (11) 2-methyltetrahydrofuran; (12) tetrahydrofuranalcohol; (13) 1,5-pentanediol; (14) furan; and (15) tetrahydrofuran.

Furfural is hydrogenated to furfurylalcohol and tetrahydrofuranalcohol. Other hydrogenation products include 2-methylfuran and 2-methyl-tetrahydrofuran (see: F.W. Lichtenthaler, S. Peters / C. R. Chimie 7 (2004) 65-90).

Furfural can also be hydrogenated to yield 1,5-pentandiol, furan or tetrahydrofuran (see: Anurag Jaswal et al., Green Chem., 2022, 24, 510ff and R. Mariscal et al., Energy Environ. Sci., 2016, 9, 1144ff and Xiaodan Li et al., ACS Catal. 2016, 6, 7621-7640).

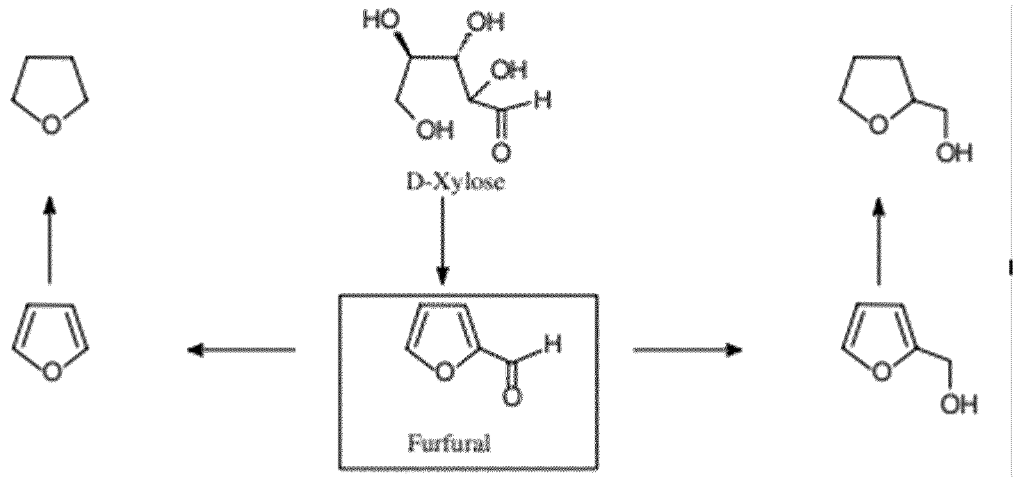


Source: F.W. Lichtenthaler, S. Peters / C. R. Chimie 7 (2004) 65-90

The inventive processes of this application and the thus synthesized chemical compounds can be used in the synthesis of surfactants: Mono-, Oligo- and polysaccharides can be reduced to sugar alcohols and other alcohols using the inventive hydrogen. Typically this is done following a chemical or enzymatic hydrolysis step in which the constituent sugar units are formed, but hydrolysis and hydrogenatation may also be carried out in one step and alternatively, the reduction may also be carried out on sugars and sugar mixtures obtained from other processes. The hydrogenation process typically involves the use of one or more catalysts, e.g. Raney Nickel, and is carried out under a Hydrogen atmosphere. The

hydrogenation yields sugar alcohols, or other reduced fragments. Examples for carrying out such reductions using the inventive hydrogen can be found in WO2008077640, EP1412083, WO2014052374, WO2017/001382, Angew. Chem. Int. Ed. 2008, 47, 8510-8513 and in the documents cited therein.

Examples:

| Reactant | Product | Conditions |
|---|---|---|
| Corn Syrup | main products monoethylene glycol, monopropylene glycol, hydroxyacetone (HA), butanedioland 1-hydroxy-2-butanone (1H2BO) | Raney Ni, sodium metatungstate, hydrogen carbonate, water, 230 °C 100 bar hydrogen |
| isomaltulose | Main products: glucopyranosyl-mannitol, glucopyranosyl-sorbitol | Nickel catalyst, 130 °C, 30 bar Hydrogen, water |
| D-Glucose | Sorbitol | Ruthenium on $SiO_2$ catalyst, water, 50 bar hydrogen, 120 °C |

Furfural, obtainable from hemicellulose biomass and also from other sustainable sources can be reacted with the inventive hydrogen. Examples for such transformations can be found in the following publications and in documents cited therein: Renewable and Sustainable Energy Reviews Volume 38 Pages 663-676, Kangyu Zhao et al. in Green Chemistry First published 16 May 2024, EP2051972.

Examples:

| Reactant | Product | Conditions |
|---|---|---|
| Furfural | Tetrahydrofurfuryl alcohol | $Pd_1Cu_{19}$-catalyst, water, 10 bar hydrogen, 50 °C |

The products resulting from the hydrogenation of Mono-, Oligo- and polysaccharides or from the hydrogenation of furfural using the inventive hydrogen can be used to manufacture surfactants, for example by forming esters or ethers with other compounds, for example with fatty acid derivatives or alkylene oxides.

The inventive products obtained by the hydrogenation of carbohydrates are characterized by the molar share of deuterium in the hydrogen bound in the products as a result of step (b) of the process according to the invention of ≤ 100 ppm, preferably in the range of from 10 to 95 ppm, more preferably in the range of from 15 to 90 ppm, most preferably in the range of from 20 to 80 ppm, especially in the range of from 30 to 75 ppm, based on the total content of said hydrogen.

Said low deuterium molar share is introduced by step (a) of the process according to the present invention. With the present invention environmentally friendly products obtained by the hydrogenation of carbohydrates and an environmentally friendly process for making the same, wherein said process uses as little fossil energy as possible, are provided.

As mentioned above, it is important that the origin of the hydrogen and down-stream compounds obtained by clean energy can be tracked in a reliable way.

Today, the majority of hydrogen is produced from fossil fuels by steam reforming of natural gas and other light hydrocarbons, partial oxidation of heavier hydrocarbons, and coal gasification. According to the invention, hydrogen obtained by electrolysis is obtained by using non-fossil energy sources. It is expected that the electrification (power generation) of fossil sources will be fully replaced by the generation of power by non-fossil resources in the near future. The downstream products based on hydrogen obtained by electrolysis can be distinguished by their deuterium molar share from amine compounds based on hydrogen prepared by petrochemical processes.

The present invention therefore relates to the use of the molar share of deuterium in downstream compounds based on hydrogen for tracing the origin, especially the energetic origin, of the hydrogen bound in the downstream compounds based on hydrogen, wherein the downstream compounds are products obtained by the hydrogenation of carbohydrates.

The present invention further relates to a process for tracing the origin, especially the energetic origin, of hydrogen bound in downstream compounds based on hydrogen by determining the molar share of deuterium in said

downstream compounds based on hydrogen, wherein the downstream compounds are products obtained by the hydrogenation of carbohydrates.

Tracing is in the meaning of the present invention synonymous with tracking.

The origin is in the meaning of the present invention the preparation method of the hydrogen employed, especially electrolysis and/or the energetic origin, i.e. non-fossil energy sources. As mentioned above, it is expected that the electrification (power generation) of fossil sources will be fully replaced by the generation of power by non-fossil resources in the near future. Hydrogen made by electrolysis is in this case hydrogen of non-fossil origin. Examples for non-fossil power sources are mentioned above.

The inventive process for tracing the origin, especially the energetic origin, of hydrogen and downstream compounds mentioned above may be employed as a single tracing (tracking) method or in combination with further tracing (tracking) methods.

Furthermore, the products according to the process described herein can be converted to a product as described on page 18, line 29 to page 26, line 29 of European patent application No. EP24164893.0.

I Hydrogen Production

Experimental Setup and Method: Electrolysis cell design and Hydrogen production conditions

1) Polymer Electrolyte Membrane / Proton Exchange Membrane Electrolysis (PEM)

Electrolysis Cell:Water electrolysis was conducted with a circular commercial PEM electrolysis cell (model ZE 200, Sylatech Analysetechnik GmbH, 0.007 $m^2$ active area). The cell stack was sealed with O-rings and wrapped with heat insulation fabric for isothermal operating conditions. A Nation® 117 standard membrane (supplier DuPont, dry thickness 180 microns) was assembled by HIAT GmbH with catalytic active coating materials iridium (19 g/$m^2$) and platinum (8 g/$m^2$). Water distribution at the anode half-cell was realized with a titanium mesh. Before the polymer electrolyte membrane, a porous transport layer with sintered titanium fiber material is used for controlled flow while a porous graphite plate was situated on the cathode-side.

Experimental Conditions: Water with controlled temperature was supplied at constant flow of 9.5 g/h to the anode compartment. The cell pressure on cathode and anode side was controlled with PC valves. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic cell-water was re-cycled, whereas on the cathode the separated water was drained. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

2) Alkaline Electrolysis Cell (AEC)

Electrolysis Cell: Alkaline water electrolysis was conducted with a circular AEC electrolysis cell (model Electro MP Cell, supplier ElectroCell Europe A/S, 0.01 $m^2$ electrode area). As electrodes Nickel 2.4068 material was used and separated by a commercial standard Zirfon Perl UTP 500 membrane (open mesh polyphenylene sulfide fabric symmetrically coated with a mixture polymer / zirconium oxide; thickness 500 microns; 0.023 $m^2$ active area; supplier Agfa-Gevaert N.V.) in zero-gap cell configuration.

Experimental Conditions: Alkaline water (32 wt% potassium hydroxide, technical standard grade) with controlled temperature was supplied at constant flow of 27.8 kg/h to the anode and cathode compartment. The cell pressure on cathode and anode side was equalized via PC valve control. Experimental conditions such as temperature and pressure settings see table. The evolved hydrogen and oxygen gas in the half-cells was separated in a two-step separator setup with an intermediate condenser cooled with 20°C cooling water. Water condensate flowed back to the separator tank. Anodic and cathodic cell-water was re-cycled. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

3) Anion Exchange Membrane / Alkaline Electrolyte Membrane Electrolysis (AEM)

Electrolysis Cell: The AEM experiments were executed in a commercial, fully automated 2.4 kW EL 4.0 cell supplied by Enapter GmbH, 10117 Berlin and a 1 wt% potassium hydroxide (standard grade) electrolyte solution.

Test station: As recommended by the supplier the EL 4.0 electrolyzer is run with a 1 wt% potassium hydroxide (technical standard grade) solution, hydrogen production at operating conditions (experimental conditions such as

temperature and pressure settings see table) was 480 l/h at approx. 400 ml water consumption. The evolved hydrogen gas was treated with desiccant dryers, ensuring complete separation of moisture in the electrolysis gas flow to yield < 0,03 wt% water in the hydrogen gas stream. Small gas samples of the dehydrated cathodic hydrogen gas were taken from the continuous flow with an auto-sampler at regular intervals to conduct hydrogen gas analytics described below.

4) Solid Oxide Electrolysis Cell (SOE)

Solid Oxide cell stacks from Elcogen- Elco Stack (Elcogen OY, Vantaa 01510 Finland), were used and a E3000 unit was operated in reverse mode at 700°C and 35A electrolyzing current; Anode functional composition due to the supplier is NiO/YSZ. Cathode is of LSC type [La(Sr)CoO3]: The principle is also described in Novel high-performance solid oxide fuel cells with bulk ionic conductance dominated thin-film electrolytes - ScienceDirect (www.sciencedirect.com/science/article/pii/S037877531201097X); D. Stover et al, Journal of Power Sources; Volume 218, 15 November 2012, Pages 157-162.

The hydrogen stream was used with no further purification/dryer. Remaining gas moisture is separated by desiccant dryers, ensuring complete separation of moisture in the electrolysis gas flow. Small gas samples of the dehydrated cathodic hydrogen gas were taken from the continuous flow with an auto-sampler at regular intervals to conduct hydrogen gas analytics described below.

The results of the hydrogen production are shown in Table 1.

Table 1

| Electrolyser type | Example I) PEM-electrolyser | Example II) PEM electrolyser | Example III) Alkaline electrolyser | Example IV) Alkaline electrolyser | Example V) AEM | Example VI) AEM | Example VII) Solid oxide |
|---|---|---|---|---|---|---|---|
| Water-type/deuterium content | VSMOW/155,76 ppm D | Selected* surface water/139 ppm D | VSMOV/155,76 ppm D | Selected* surface water/139ppm D | VSMOW/155,76 ppm D | Selected* surface water/139 ppm D | Selected* surface water/139 ppm D |
| Current density [A/cm2] | 1.35 | 1.35 | 0.8 | 0.8 | 1.6 | 1.6 | 0.78 |
| Applied voltage [V] | 1.9 | 1.9 | 2.15 | 2.15 | 1.9 | 1.9 | 1.35 |
| Operating temperature [°C] | 68 | 68 | 81 | 81 | 45 | 45 | 710 |
| Cell pressure [bar] | 23 | 23 | 25 | 25 | 35 | 35 | 1.3 |
| Voltage efficiency [%] | 57 | 57 | 53 | 53 | 66 | 66 | 81 |
| Electrical efficiency [kWh/kg H2] | 52 | 52 | 48.5 | 48.6 | 69 | 69 | 47 |
| Deuterium content in hydrogen [ppm] | 86 | 77 | 95 | 87 | 74 | 66 | 38 |
| Isar river surface water (Munich) collected in winter season January 2021. | | | | | | | |

Experimental Setup and Method: "D content (Deuterium content) in samples"

The following method descriptions apply for determination of the molar share of deuterium based on the total hydrogen content (Deuterium content) of gas and liquid samples. The isotopic H/D-share analysis is based on mass spectroscopy. Two different methods are used: method A for gas samples and method B for liquid samples.

For determination of the "D content in gas and liquid samples" it is of crucial importance not to contaminate the samples e.g. with ambient humidity or other ambient components containing hydrogen or deuterium. Therefore gas-tight materials and sealings must be used with clean sample containers to avoid any cross-contamination. Therefore, before filling and sealing a sample container it must be flushed at least 20 times the sample container volume with the gas or liquid stream to be analyzed. The same is valid for the experimental setup of the gas sampler and mass spectrometer. Utmost care must be taken to avoid cross-contamination e.g. via condensation of humidity. The analytical setup from sampling to mass spectrometry is validated with known reference samples.

Method A) Gas samples

Total Deuterium from HD and $D_2$ in hydrogen gas samples was determined via ultra-high resolution quadrupole mass spectrometry using a Hiden DLS-20 (Hiden Analytical Ltd., Warrington, Cheshire, UK) analyzer setup. The general method setup is described in C.C. Klepper, T.M. Biewer, U. Kruezi, S. Vartanian, D. Douai, D.L. Hillis, C. Marcus, Extending helium partial pressure measurement technology to JET DTE2 and ITER; Rev. Sci. Instrum., 87 (11) (2016); doi: 10.1063/1.4963713. For the hydrogen gas samples the threshold ionization mass spectrometry mode (TIMS) was used as described in S. Davies, J.A. Rees, D.L. Seymour; Threshold ionization mass spectrometry (TIMS); A complementary quantitative technique to conventional mass resolved mass spectrometry; Vacuum, 101 (2014), pp. 416-422; doi: 10.1016/j.vacuum.2013.06.004. Sensitivity is +/-1 ppm.

Method B) Liquid samples

Analysis of liquid and solid samples (carbohydrates and products obtained by the hydrogenation of carbohydrates) was executed via isotope ratio monitoring gas chromatography/mass spectrometry (IRMS). Therefore a DELTA V PLUS CF-IRMS mass spectrometer was used. This mass spectrometer with magnetic sector with continuous flux DELTA V PLUS CF-IRMS is used to measure the isotopic ratio of D/H.

Measurement of D/H in a continuous He-flow mode needs the complete removal of low energy 4 He+ ions from the HD+ ion beam at m/z 3). The method is described in RAPID COMMUNICATIONS IN MASS SPECTROMETRY Rapid Commun. Mass Spectrom. 13, 1226-1230 (1999), W.A. Brandt et al. Sensitivity is within +/-3 ppm.

Method C) Analysis of isotope ratios in starting materials and products

Analysis of isotope ratios in starting materials and products was furthermore executed via SNIF-NMR related methods ("site-specific natural isotope fractionation studied by nuclear magnetic resonance") using high resolution $^2$H-NMR (Bruker Avance NEO 600 MHz NMR Spectrometer and Bruker Avance III HD 700 MHz NMR Spectrometer both equipped with TCI probes).

Principles of this technology are described in Gérard J. Martin, Serge Akoka, and Maryvonne L. Martin; SNIF-NMR Part 1: Principles; in Graham A. Webb (ed.), Modern Magnetic Resonance, Springer (2008) pp 1651-1658 as well as Maryvonne Martin, Benli Zhang, and Gérard J. Martin; SNIF-NMR Part 2: Isotope Ratios as Tracers of Chemical and Biochemical Mechanistic Pathways; in Graham A. Webb (ed.), Modern Magnetic Resonance, Springer (2008) pp 1659-1667 and Gérard J. Martin, Maryvonne L. Martin and Gérald Remaud; SNIF-NMR Part 3: From Mechanistic Affiliation to Origin Inference; in Graham A. Webb (ed.), Modern Magnetic Resonance, Springer (2008) pp 1669-1680.

II Products obtained by the hydrogenation of carbohydrates

Products obtained by the hydrogenation of carbohydrates are prepared in industrial scale synthesis processes as described above.

## Claims

**1.** A process for the preparation of a hydrogenation product of a carbohydrate or furfural comprising the following steps:

(a) providing hydrogen with a molar share of deuterium ≤ 100 ppm, based on the total hydrogen content, by

electrolysis of water using electrical power generated at least in part from non-fossil energy,
(b) at least partially hydrogenating a carbohydrate or furfural to form the corresponding hydrogenation product of the carbohydrate or furfural.

**2.** The process of claim 1, wherein the electrical power is generated from wind power, solar energy, biomass, hydropower and geothermal energy.

**3.** The process according to claim 1 or 2, wherein hydrogen is provided by polymer electrolyte membrane water electrolysis.

**4.** The process according to claim 3, wherein hydrogen is provided by proton exchange membrane water electrolysis (PEMWE) or anion exchange membrane water electrolysis (AEMWE).

**5.** The process according to any one of claims 1 to 4, wherein the molar share of deuterium in the hydrogen provided in step (a) is in the range of from 10 to 95 ppm, preferably in the range of from 10 to 90 ppm, more preferably in the range of from 20 to 80 ppm, and most preferably in the range of from 30 to 75 ppm, based on the total hydrogen content.

**6.** The process according to any one of claims 1 to 5, wherein step (b) is carried out as heterogeneously or homogeneously catalyzed reaction.

**7.** The process according to any one of claims 1 to 6, wherein the carbohydrate to be hydrogenated in step (b) contains a total of from 1 to 20 monosaccharide units or a total of from 3 to 120 carbon atoms.

**8.** The process according to claim 7, wherein the carbohydrates to be hydrogenated in step (b) contain a total of from 1 to 10 monosaccharide units or a total of from 3 to 60 carbon atoms.

**9.** The process according to claim 8, wherein the carbohydrates to be hydrogenated in step (b) contain a total of from 1 to 5 monosaccharide units or a total of from 3 to 30 carbon atoms.

**10.** The process according to any one of claims 1 to 9, wherein the carbohydrate to be hydrogenated in step (b) is a monosaccharide or a disaccharide and is hydrogenated to give the corresponding sugar alcohol.

**11.** The process according to any one of claims 1 to 10, wherein the carbohydrate is partially hydrogenated in step (b).

**12.** The process according to any one of claims 1 to 11, wherein the hydrogenation of the carbohydrate is selected from

(1) hydrogenation of glucose to sorbitol;
(2) hydrogenation of mannose to mannitol;
(3) hydrogenation of fructose to mannitol;
(4) hydrogenation of xylose to xylitol;
(5) hydrogenation of isomaltulose to isomalt;
(6) hydrogenation of maltose to maltitol;
(7) hydrogenation of lactose to lactitol;
(8) hydrogenation of starch hydrolysates to Hydrogenated starch hydrolysates.

**13.** The process according to any one of claims 1 to 11, wherein the hydrogenation of furfural is selected from

(9) hydrogenation of furfural to furfurylalcohol;
(10) hydrogenation of furfural to 2-methylfuran;
(11) hydrogenation of furfural to 2-methyltetrahydrofuran;
(12) hydrogenation of furfural to tetrahydrofuranalcohol;
(13) hydrogenation of furfural to 1,5-pentanediol;
(14) hydrogenation of furfural to furan;
(15) hydrogenation of furfural to tetrahydrofuran.

**14.** Hydrogenation products of carbohydrates or furfural, obtainable by the process according to any one of claims 1 to 13.

**15.** Hydrogenation products of carbohydrates or furfural according to claim 13, wherein the molar share of deuterium in

the hydrogen bound in the hydrogenation product as a result of step (b) of the process according to any one of claims 1 to 12 is ≤ 100 ppm, preferably in the range of from 10 to 95 ppm, more preferably in the range of from 15 to 90 ppm, most preferably in the range of from 20 to 80 ppm, especially in the range of from 30 to 75 ppm, based on the total content of said hydrogen.

**16.** The use of the molar share of deuterium in downstream compounds based on hydrogen for tracing the origin, especially the energetic origin, of hydrogen bound in the downstream compounds based on hydrogen, wherein the downstream compounds are products obtained by the hydrogenation of carbohydrates or furfural.

**17.** A process for tracing the origin, especially the energetic origin, of hydrogen bound in downstream compounds based on hydrogen by determining the molar share of deuterium in said downstream compounds based on hydrogen, wherein the downstream compounds are products obtained by the hydrogenation of carbohydrates of furfural.

Europäisches Patentamt
European Patent Office
Office européen des brevets

# EUROPEAN SEARCH REPORT

Application Number
EP 24 20 7976

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | HARADA, K. ET AL.: "Effects of water transport on deuterium isotope separation during polymer electrolyte membrane water electrolysis", INTERNATIONAL JOURNAL OF HYDROGEN ENERGY, vol. 45, no. 56, 2020, pages 31389-31395, XP086328555, ISSN: 0360-3199, DOI: 10.1016/J.IJHYDENE.2020.08.256 [retrieved on 2020-09-26] * abstract * | 1-17 | INV. C07D307/06 C07D307/08 C07D307/12 C07D307/36 C07D307/44 C07C29/132 C07C31/18 C07C31/20 C07C31/26 C07H15/04 C01B3/02 C25B1/04 |
| Y,D | SATO, H. ET AL.: "Effect of water stoichiometry on deuterium isotope separation by anion exchange membrane water electrolysis", INTERNATIONAL JOURNAL OF HYDROGEN ENERGY, vol. 46, no. 68, 2021, pages 33689-33695, XP086784907, ISSN: 0360-3199, DOI: 10.1016/J.IJHYDENE.2021.07.202 [retrieved on 2021-08-20] * abstract * | 1-17 | |
| | -/-- | | TECHNICAL FIELDS SEARCHED (IPC) |
| | | | C07D C07C C07H C01B C25B |

The present search report has been drawn up for all claims

| Place of search | Date of completion of the search | Examiner |
|---|---|---|
| Munich | 27 November 2024 | Kiernan, Andrea |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 24 20 7976

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | OÑA, J.P. ET AL.: "Electrocatalytic Hydrogenation of Glucose and Xylose on Electrochemically Roughened Metal Catalysts", ACS CATALYSIS , vol. 13, no. 21 23 October 2023 (2023-10-23), pages 14300-14313, XP093144209, ISSN: 2155-5435, DOI: 10.1021/acscatal.3c04043 Retrieved from the Internet: URL:https://pubs.acs.org/doi/pdf/10.1021/acscatal.3c04043<br>* abstract *<br>* Introduction; page 14300, paragraph 1. *<br>* page 14302 *<br>----- | 1-12,14, 15<br><br>1-17 | |
| X<br><br>Y | ZHANG, L. ET AL.: "A review of thermal catalytic and electrochemical hydrogenation approaches for converting biomass-derived compounds to high-value chemicals and fuels", FUEL PROCESSING TECHNOLOGY, vol. 226, 2021, XP086880854, ISSN: 0378-3820, DOI: 10.1016/J.FUPROC.2021.107097 [retrieved on 2021-11-19]<br>* abstract *<br>* page 2, column 2 *<br>* page 12; table 5 *<br>* Scheme 3; page 16 - page 18; tables 8, 9 *<br>----- | 1-15<br><br>1-17 | TECHNICAL FIELDS SEARCHED (IPC) |

-/--

The present search report has been drawn up for all claims

2

| Place of search | Date of completion of the search | Examiner |
|---|---|---|
| Munich | 27 November 2024 | Kiernan, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 20 7976

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | VEDOVATO, V. ET AL.: "Electrosynthesis of Biobased Chemicals Using Carbohydrates as a Feedstock", MOLECULES, vol. 25, no. 16, 2020, page 3712, XP093145916, ISSN: 1433-1373, DOI: 10.3390/molecules25163712 | 1,2, 5-12,14, 15 | |
| Y | * abstract * * page 25 - page 30 * * page 27, last paragraph; table 9 * * Scheme 21; page 26 * * Scheme 23; page 29 * * page 30; table 11 * ----- | 1-17 | |
| | | | TECHNICAL FIELDS SEARCHED (IPC) |

The present search report has been drawn up for all claims

2

| Place of search | Date of completion of the search | Examiner |
|---|---|---|
| Munich | 27 November 2024 | Kiernan, Andrea |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 201760922 A
- WO 2008077640 A
- EP 1412083 A
- WO 2014052374 A
- WO 2017001382 A
- EP 2051972 A
- EP 24164893


### Non-patent literature cited in the description


- **S. KUMAR** ; **V. HIMABINDU**. *Material Science for Energy Technologies*, 2019, vol. 2, 4442-4454
- **H. A. MILLER et al.** *Sustainable Energy Fuels*, 2020, vol. 4, 2114-2133
- **K. HARADA et al.** *International Journal of Hydrogen Energy*, 2020, vol. 45, 31389-31395
- **H. SATO et al.** *International Journal of Hydrogen Energy*, 2021, vol. 46, 33 689-33 695
- Sugar Alcohols. **H. SCHIWECK et al.** Ullmann's Encyclopedia of Industrial Chemistry. Wiley-VCH, 2012
- **RUPPERT, A. et al.** Hydrogenolysis Goes Bio: From Carbohydrates and Sugar Alcohols to Platform Chemicals. *Angewandte Chemie International Edition*, 2012, vol. 51 (11), 2564-2601
- Hydrogenation and Dehydrogenation. **C.STOCK**. Ullmann's Encyclopedia of Industrial Chemistry. 2023
- Sorbitol and Mannitol. **KEARSLEY MW, DEIS RC**. Sweeteners and Sugar Alternatives in Food Technology. Wiley-Blackwell., 2006, 249-261
- **ALTSCHUL** ; **AARON M**. Low-Calorie Foods Handbook. CRC Press, 12 March 1993
- **CAI** ; **CHARLES M et al.** Integrated furfural production as a renewable fuel and chemical platform from lignocellulosic biomass. *Journal of Chemical Technology & Biotechnology*, 2014, vol. 89, 2-10
- **ANURAG JASWAL et al.** *Green Chem.*, 2022, vol. 24, 510
- **R. MARISCAL et al.** *Energy Environ. Sci.*, 2016, vol. 9, 1144
- **XIAODAN LI et al.** *ACS Catal.*, 2016, vol. 6, 7621-7640
- *Angew. Chem. Int. Ed.*, 2008, vol. 47, 8510-8513
- *Renewable and Sustainable Energy Reviews*, vol. 38, 663-676
- **KANGYU ZHAO et al.** *Green Chemistry*, 16 May 2024
- **D. STOVER et al.** *Journal of Power Sources*, 15 November 2012, vol. 218, 157-162
- **C.C. KLEPPER** ; **T.M. BIEWER** ; **U. KRUEZI** ; **S. VARTANIAN** ; **D. DOUAI** ; **D.L. HILLIS** ; **C. MARCUS**. Extending helium partial pressure measurement technology to JET DTE2 and ITER. *Rev. Sci. Instrum.*, 2016, vol. 87 (11)
- **S. DAVIES** ; **J.A. REES** ; **D.L. SEYMOUR**. Threshold ionization mass spectrometry (TIMS). *Vacuum*, 2014, vol. 101, 416-422
- RAPID COMMUNICATIONS IN MASS SPECTROMETRY. Rapid Commun. Mass Spectrom.. 1999, vol. 13, 1226-1230
- **W.A. BRANDT et al.** *Sensitivity is within +/-3 ppm*
- SNIF-NMR Part 1: Principles. **GÉRARD J. MARTIN** ; **SERGE AKOKA** ; **MARYVONNE L. MARTIN**. Modern Magnetic Resonance. Springer, 2008, 1651-1658
- SNIF-NMR Part 2: Isotope Ratios as Tracers of Chemical and Biochemical Mechanistic Pathways. **MARYVONNE MARTIN** ; **BENLI ZHANG** ; **GÉRARD J. MARTIN**. Modern Magnetic Resonance. Springer, 2008, 1659-1667
- SNIF-NMR Part 3: From Mechanistic Affiliation to Origin Inference. **GÉRARD J. MARTIN** ; **MARYVONNE L. MARTIN** ; **GÉRALD REMAUD**. Modern Magnetic Resonance. Springer, 2008